# EUROPEAN PATENT APPLICATION

(11) **EP 1 949 919 A2**
(43) Date of publication of application: **30.07.2008**
(21) Application number: 07253818.4
(22) Date of filing: 26.09.2007
(51) Int. Cl.: A61L 27/38

(54) **Multilayered composite for organ augmentation and repair**

(30) Priority: 29.09.2006 US 847922 P
(71) Applicant: Johnson & Johnson Regenerative Therapeutics, LLC, Raynham, MA 02767 (US)
(72) Inventor: Keeley, Daniel, Boston, MA 02215 (US); Shetty, Dhanuraj, Somerset, NJ 08873 (US); Dhanaraj, Sridevi, Raritan, NJ 08869 (US); Wang, Ziwei, Monroe Twp, NJ 08831 (US)
(74) Representative: Belcher, Simon James

(57) **Abstract**

A device for augmenting or regenerating tissue comprises first and second layers of biocompatible, biodegradable scaffolding and at least one layer of autologous tissue disposed between and substantially in contact with the first and second scaffolding layers.

## Description

The present invention relates to devices and methods for tissue augmentation or regeneration. More specifically, the present invention provides for a composite of biocompatible scaffold and autologous tissue, suitable for implantation in a hollow organ or skin.

Regenerative medicine strives to treat disease and restore human tissues by prompting the body to autonomously regenerate damaged tissue. Tissue engineered implants may prompt such regeneration by providing structure and media for cell growth, and may enable direct transplantation of healthy tissues into a damaged-tissue environment.

Many of these new therapies require implantable biocompatible and biodegradable scaffolds for use both in vitro and in vivo. These scaffolds may augment healing through tissue infiltration or by providing suitable means of cell attachment and proliferation. Also, these scaffolds may be seeded with cells and manufactured in such a way that chemical, mechanical, and cellular stimuli are optimized. Despite advances made in this field in recent years, there remains a need for improved approaches to tissue scaffolding, particularly in the area involving the skin, or hollow organs such as the bladder, urethra, jejunum, esophagus, or trachea.

In one aspect, the present invention provides an implant for tissue augmentation and regeneration in hollow organs, comprising a biocompatible, biodegradable scaffold that sandwiches autologous cells or tissue.

Preferably, two or more layers of biocompatible scaffolding and autologous tissue or cells provide a means to promote growth of tissues. In an aspect of the invention, the tissue contains more than one type of cell.

The device may hold the cellular component in place and also include a means of fixation, such as a suture.

The device of the invention can be used in a method of using an implant to augment tissue regeneration in a hollow organ.

The cellularized implants of the invention can be used to patch holes, repair areas of damaged tissue, or increase the surface area of tissues in a hollow organ.

The invention provides a method for augmenting or regenerating organ tissue comprising:
(a) obtaining a first and second layer of biocompatible, biodegradable scaffolding;
(b) obtaining a sample of autologous tissue;
(c) sandwiching said autologous tissue in between and in substantial contact with said first and second scaffolding layers; and
(d) fixing said scaffold tissue sandwich to said organ tissue such that one scaffold layer is positioned on the exterior of said organ, one scaffold layer is positioned on the interior of the organ, and the autologous layer is aligned with the organ tissue.

The method can include the step of fixing said scaffold-tissue composite in place.

The invention also provides a device which is produced by the method defined above.

The invention is described by way of example with reference to the accompanying drawings, in which:
Figure 1 depicts an embodiment of the present invention in which autologous cellular tissue is sandwiched between layers of biocompatible, biodegradable scaffolding.
Figure 2 depicts an embodiment of the present invention in which isolated cells are sandwiched between layers of biocompatible, biodegradable scaffolding.
Figure 3 depicts an embodiment of the present invention in which dissected tissue is removed from a hollow organ, placed between layers of biocompatible, biodegradable scaffolding, and implanted into a hollow organ to provide a tissue patch that replaces or increases the surface area of the hollow organ.
Figure 4 depicts an embodiment of the present invention in which a composite comprising autologous cellular tissue and biocompatible, biodegradable scaffolding is sutured into native tissue.

Other than in the operating examples, or where otherwise indicated, all numbers expressing quantities of ingredients or reaction conditions used herein should be understood as modified in all instances by the term "about." The term "about" when used in connection with percentages may mean ± 1%.

The present invention provides for a layered composite device and method to use the device such that the invention assists with tissue augmentation and regeneration. More specifically, multiple layers of biocompatible scaffolding and autologous tissue may provide the means to promote growth of tissues containing more than one type of cell. When utilized, these cellularized patches will be able to fill holes, repair areas of damage, or increase the surface area of tissues in hollow organs. The device may not only hold the autologous cellular and tissue in place, but may also contain the means for fixing the implant.

Recent publications have discussed various scaffolding approaches for reconstruction of skeletal or tract tissues. For example, US-A-2005/0154458 refers to an "active bio layer" such as hyaluronic acid, capable of interacting with stem cells from bone marrow, sandwiched between opposed surfaces of biomaterials or synthetic polymer materials, for skeletomuscular applications. The present invention does not require an "active bio layer" and is not limited to such skeletomuscular applications. US-A-2004/0225247 refers to a tissue patch having a protective layer for repairing an alimentary tract lesion. The present invention has no such "protective liner," nor is it limited to such lesions. US-A-2005/0272153 refers to a metal coated scaffold (and thus non-degradable) with biocompatible material (but not cells or tissue) for implantation into or in place of bone. Finally, US-6143293 refers to a stack of cell-seeded hydroxyapatite scaffolds for use as a 3-dimensional void filler for use in, for example, bone. The present invention is not directed to such void filler uses.

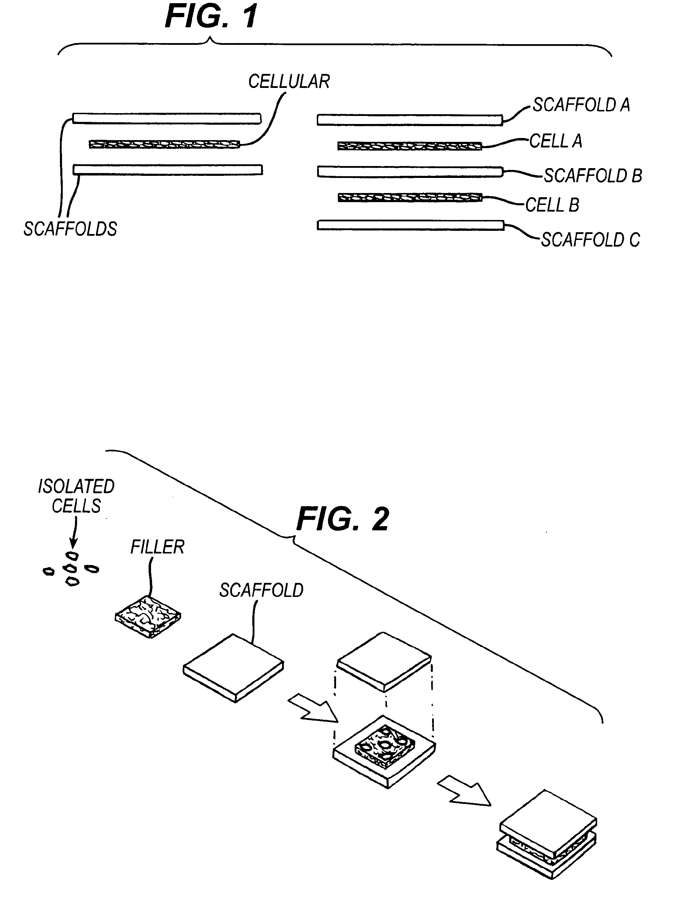

Preferably, each composite contains at least two scaffolding layers and at least one cellular layer. See, for example, Figure 1. These layers may be placed upon each other in various fashions to form an implant system with a living component. The scaffolding layers may be porous or non-porous, and may be made from natural polymers, synthetic polymers, bioactive glass, hydrogel, or any biocompatible material that may be manufactured in a flat sheet. One or more sheets may have agents or bioactive agents incorporated within or disposed upon their matrix.

The scaffold layers of the present invention are bioabsorbable, meaning that they are biocompatible and biodegradable. Biocompatible refers to materials which do not have toxic or injurious effects on biological functions. Biodegradable refers to material that can be absorbed or degraded in a patient's body. Representative materials for forming the biocompatible structure include natural or synthetic polymers, such as, for example, collagen, poly(alpha esters) such as poly(lactic acid), poly(glycolic acid), polyorthoesters and polyanhydrides and their copolymers, which degrade by hydrolysis at a controlled rate and are reabsorbed. These materials provide the maximum control of degradability, manageability, size and configuration. Other biodegradable polymer materials include polyglycolic acid and polyglactin, for example as disclosed in US-5514181.

Other biodegradable materials include cellulose ether, cellulose, cellulosic ester, fluorinated polyethylene, phenolic, poly-4-methylpentene, polyacrylonitrile, polyamide, polyamideimide, polyacrylate, polybenzoxazole, polycarbonate, polycyanoarylether, polyester, polyestercarbonate, polyether, polyetheretherketone, polyetherimide, polyetherketone, polyethersulphone, polyethylene, polyfluoroolefin, polyimide, polyolefin, polyoxadiazole, polyphenylene oxide, polyphenylene sulphide, polypropylene, polystyrene, polysulphide, polysulphone, polytetrafluoroethylene, polythioether, polytriazole, polyurethane, polyvinyl, polyvinylidene fluoride, regenerated cellulose, silicone, urea-formaldehyde, or copolymers or physical blends of these materials.

An example of a biocompatible, biodegradable polymer suitable for the instant invention is the polyglactin which is available from Novartis-Ethicon under the trade mark Vicryl, especially polyglactin 910 which is a 90:10 copolymer of glycolide and lactide, derived respectively from glycolic and lactic acids.

Regarding the use of ceramics as material for scaffold formation, a bioactive glass such as that available from NovaBone Products LLC under the trade mark BioGlass can be modified with a poly(lactic co-glycolic acid) polymer matrix, for example as disclosed in US-6328990. "Bioactive" means that the material has the ability to interact or bind to living tissue.

Alternatively, hydrogels such as alginate-RGD may be used. Alginates are seaweed-derived copolymers for which the rigidity of the hydrogel may be controlled by crosslinking its glucuronate residues with, e.g., calcium or adipic dihydrazide. Alginate may further be modified with cell-adhesive peptides such as Arg-Gly-Asp (RDG) peptide to promote cellular attachment to the scaffold layer. See, e.g., Wong et al., 570 Science 119-33 (2004); Das & Hollister, Tissue Engineering Scaffolds in Encyclopaedia of Mats: Sci & Tech. 1-7 (Elsevier Sci., Ltd. 2003).

Further regarding the scaffold layer, an optional pharmaceutical or bioactive agent may be incorporated into the scaffolding. The variety of different pharmaceuticals that can be used in conjunction with the scaffolds of the present invention is vast. Such pharmaceuticals or agents will in general be selected according to the tissue or organ being reconstructed or augmented, to ensure that appropriate new tissue is formed in the engrafted organ or tissue (for examples of such additives for use in promoting bone healing, see, e.g., Kirker-Head, 24(5) C. A. Vet. Surg. 408-19 (1995)). Common pharmaceuticals and bioactive agents which may be administered via the pharmaceutical compositions of the invention include, without limitation: anti-infectives such as antibiotics and antiviral agents; chemotherapeutic agents; anti-rejection agents; analgesics and analgesic combinations; anti-inflammatory agents; hormones such as steroids; growth factors; and other naturally derived or genetically engineered proteins, polysaccharides, glycoproteins, or lipoproteins.

Scaffolds containing these materials may be formulated by mixing one or more agents with the material used to make the scaffold. Alternatively, an agent could be coated onto the scaffold, preferably with a pharmaceutically acceptable carrier. Any pharmaceutical carrier can be used that does not dissolve or react with the scaffold. The pharmaceutical agents may be present as a liquid, a finely divided solid, or any other appropriate physical form. Typically, but optionally, they will include one or more additives, such as diluents, carriers, excipients, stabilizers or the like. Additionally, such optional agent or bioactive agent may be added separately (i.e., not manufactured into the scaffold matrix. For example, a bioactive agent such as fibrin may be added to the tissue or cell layer, and may serve as a bioactive glue between the cell or tissue layer and the scaffold layer(s).

A composite can include distinct scaffold layers having different physiochemical properties. For example, it is known that chemical, topographical, and mechanical cues affect cellular responses at the cell-biomaterial interface. See Wong et al., (2004). Moreover, considerations of mechanical strength in maintaining rigidity relating to a particular organ's structure and placement may suggest using a particular scaffold layer in that context. Hence, for example, a composite according to the present invention may comprise a rigid scaffold layer that provides mechanical strength in one layer, and second scaffold layer that promotes cellular ingrowth. Alternatively, different layers might be used to promote the growth of distinct cell types found in complex tissues. For example, one scaffold layer might promote the growth of cartilage, while a second scaffold layer promotes the growth of bone. Or, for example, one scaffold layer might have porosity that will foster muscle-cell infiltration while another layer might have porosity that would exclude larger cells and allow only smaller cell infiltration. Alternatively, the different scaffold layers may comprise polymers that degrade at different rates such that one layer degrades before the other. For example, gamma-irradiated PLGA degrades faster and might be used in one layer (e.g., on the inner surface of a hollow organ), while polyethylene oxide degrades more slowly and might be used in another layer (e.g., on the exterior surface of a hollow organ).

The living component of the present invention may be autologous cells or autologous tissue, obtained by any number of techniques well-known in the art. For example, during surgery a tissue sample may be obtained and simply placed on a scaffolding layer. Alternatively, tissues containing more than one cell type may be separated, for example with a scalpel, into substantially distinct tissue samples. One or more of the separated tissue samples may then be used with the scaffolding, or the separated tissues may be cellularized before placement on the scaffold. Such cellularization techniques, such as mincing or treating with appropriate cellularizing agents, are known in the art.

Alternatively, the tissue or cellularized (cell) sample may be treated in vitro before being placed on the scaffold layer. For example, cells (such as autologous cells) can be cultured in vitro to increase the number of cells available for seeding on the scaffold(s). The use of allogenic cells, and more preferably autologous cells, is preferred to prevent tissue rejection. In certain embodiments, chimeric cells, or cells from a transgenic animal, can be seeded onto the polymeric matrix. Cells can also be transfected prior to seeding with genetic material. Useful genetic material may be, for example, genetic sequences which are capable of reducing or eliminating an immune response in the host. For example, the expression of cell surface antigens such as class I and class II histocompatibility antigens may be suppressed. This may allow the transplanted cells to have reduced chance of rejection by the host. In addition, transfection could also be used for gene delivery. Urothelial and muscle cells could be transfected with specific genes prior to polymer seeding. The cell-polymer construct could carry genetic information required for the long term survival of the host or the tissue engineered neo-organ.

The composite of the present invention may be useful in treating organs. In particular, hollow organs, such as bladder, urethra, jejunum, esophagus, trachea, colon, and stomach may benefit from placement of the present composite as a "patch" in an area requiring tissue augmentation or regeneration. For example, regarding the bladder, if an area of the bladder is missing due to congenital defect or has been lost due to disease, injury or surgery (e.g., partial cystectomy), the patient may benefit from having the bladder area increased or restored to the original size as the particulars of the case allows.

Sheets of scaffold materials can be provided in a sterile form such that the physician, or other member of the surgical team, may cut the size of the particular scaffold to a size as required by the instance at hand. Multiple types of scaffold with desired physiochemical properties (as discussed above) may be provided in the same or in different packages.

The present invention allows for placement of the composite in a hollow organ such that one exterior scaffold layer may be seated upon the outside surface of the organ and the opposite exterior scaffold layer may be seated upon the inside of the organ. In such arrangement, the interior composite layers, comprising at least one tissue or cell layer (and optionally additional tissue or cell layer(s) that may or may not be further separated by additional scaffold layer(s)), to be aligned with and adjacent to the hollow organ tissue layer. So, for example, regarding the bladder, one exterior scaffold layer would rest on the serosal layer *(tunica seros),* and the opposite exterior scaffold layer would rest on the urotheliuem. Tissue layers might include, for example, detrusor *(tunica muscularis)* and lamina propria, each tissue layer positioned within the composite such that they may be aligned with the native organ tissue upon implantation. The composite is then fixed in place with, for example, suture. Such placement facilitates vascularization and cell organization as the composite integrates into the organ.

While reference is made herein to augmentation of bladder according to the invention, it will be understood that the methods and materials of the invention are useful for tissue reconstruction or augmentation of a variety of tissues and organs in a subject. Thus, for example, organs or tissues such as bladder, ureter, urethra, renal pelvis, and the like, can be augmented or repaired with polymeric scaffolds seeded with cells. The materials and methods of the invention further can be applied to the reconstruction or augmentation of vascular tissue (see, e.g., Zdrahala, 10(4) J Biomater. Appl. 309-29 (1996)), intestinal tissues, stomach (see, e.g., Laurencin et al., 30(2) J Biomed Mater. Res. 133-38 (1996)), and the like. The patient to be treated may be of any species of mammals such as a dog, cat, pig, horse, cow, or human, in need of reconstruction, repair, or augmentation of a tissue.

Living cells can be sandwiched between the scaffolding layers and substantially cover the surface area of the scaffold material. See, for example, Figure 2. These cells may be isolated through chemical digestion of their tissue matrix, or may be retained in their matrix and minced. Biocompatible filler material can be introduced to the cells to increase the relative surface area covered. In that instance, although initial cell density may have decreased, cell proliferation may eventually produce tissue covering the entire surface area. Different cell types can be isolated and layered on top of each other to ease the formation of complex tissues. These layers may, optionally, have scaffolding placed between them.

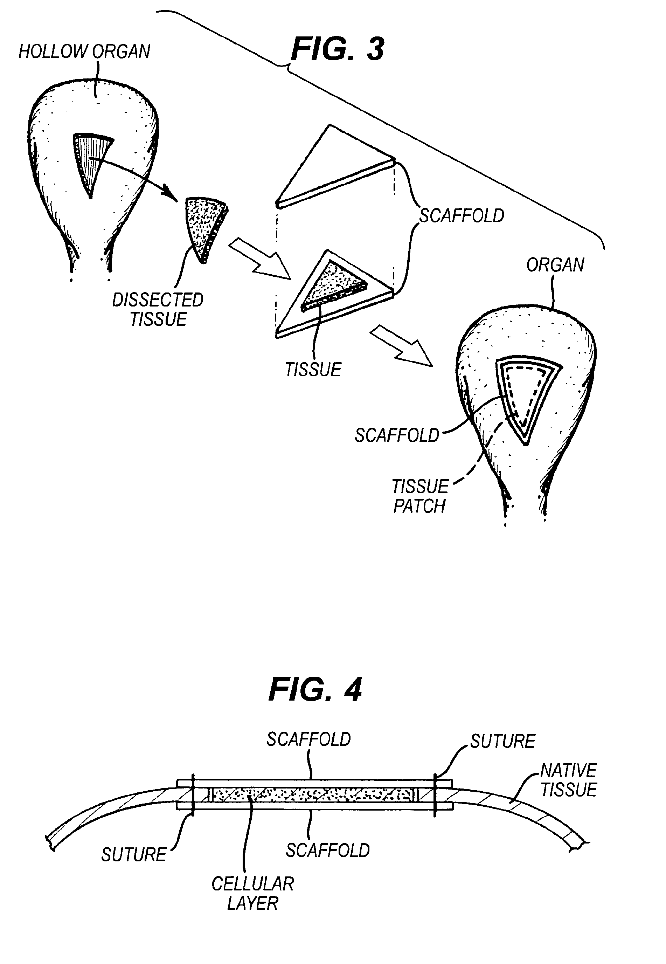

Dissected tissue can be left in its natural state and fixed between scaffold layers. See, for example, Figure 3. Between the resected tissue and the unmodified tissue, a volume of filler such as fibrin may be introduced to keep the composite structure in place.

The patch of the invention can be used in a method of treating hollow organ tissue. using the patch of the present invention. For example, as shown in Figure 3 and Figure 4, during patch fixation native tissue will be placed adjacent to the autologous cell layer and between the top and bottom scaffolds. In one aspect of the invention, the scaffold not covered with cells or tissue provides a place for suturing and attachment of the patch to the hollow organ.

### EXAMPLES

Example 1. 90/10 PGA/PLA & Small Intestine Submucosa (SIS) seeded with cells. Urothelium cells and smooth muscle cells (SMC) were isolated from porcine bladder and cultured in a humidified incubator at 37°C, 5% carbon dioxide and 95% air for one week until the cells reached 85% confluency. Porcine bladder smooth muscle cells were statically seeded at a density of 2 × 10⁶ cells/scaffold on to 90/10 PGA/PLA 11 × 7 mm scaffold discs. The SIS scaffolds were similarly prepared and seeded with porcine bladder urothelium cells at a density of 2 × 10⁶ cells/scaffold. The scaffolds were incubated in a humidified incubator at 37°C for 2 hours, after which the 90/10 PGA/PLA and SIS scaffolds were sutured together with 4-0 VICRYL coated suture (Ethicon) with both the cell-seeded surfaces placed internally and in contact with each other. The cell-seeded scaffolds were then cultured with 50% Keratinocyte-SFM medium (Invitrogen Co) and 50% SMC medium (Cambrex). After 2 weeks, the scaffolds were evaluated by histology (H&E, alpha smooth muscle actin stain, Cytokeratin-7). Both the urothelium and smooth muscle cells were retained in their respective scaffolds and retained their phenotypes as evidenced by immuno-staining.

Example 2. Coated and Uncoated 90/10 PGA/PLA scaffolds seeded with cells. Coated 90/10 PGA/PLA scaffolds were prepared by dipping the scaffolds in a 5% solution of 50/50 PGA/PLA to increase the stiffness of the scaffolds. Urothelium cells and smooth muscle cells were isolated from porcine bladder and cultured as described in example 1. Urothelium cells were loaded onto the coated 90/10 PGA/PLA scaffolds with a cell density of 2 × 10⁶ cells/scaffold. Uncoated 90/10 PGA/PLA non-woven scaffolds were seeded with porcine bladder smooth muscle cells. The cell-seeded scaffolds were incubated as in example 1, and after 2 hours incubation the cell seeded scaffolds were sutured together and cultured as described in example 1. After two weeks the scaffolds were evaluated by histology (H&E, alpha smooth muscle actin stain, Cytokeratin-7). Both the urothelium and smooth muscle cells were retained in their respective scaffolds and retained their phenotypes as evidenced by immuno-staining.

Example 3. Minced Tissue
A small 2 cm-by-2 cm piece of tissue is excised from a normal healthy bladder. The smooth muscle cell layer is then removed from the urothelial cell layer using a scalpel. This creates two distinct tissue samples for mincing. Each sample is then processed under sterile conditions to create a suspension having at least one minced, or finely divided, tissue particle.

The particle size and shape of each tissue fragment may vary. For example, the tissue size can range from about 0.1 mm³ and 3 mm³, or in the range of 0.5 mm³ and 1 mm³, or in the range of 2 mm³ and 3 mm³, or less than about 1 mm³. The shape of the tissue fragments can include, for example, slivers, strips, flakes, or cubes.

Each sample of tissue is subsequently spread on a separate 3 cm-by-3 cm square Polyglactin 910 scaffold (300 mg/cc, 1 mm thick), leaving bare 0.5 cm around the scaffold perimeter. This results in two scaffolds with two different tissue types. Fibrin glue is spread on a single scaffold and the two scaffolds are sandwiched into a five-layer composite (scaffold, minced tissue type A, fibrin, minced tissue type B, scaffold).

Larger cuts are made in the patient's bladder in a shape that eases the placement of the implant. Each scaffold is fixed by passing sutures through a layer of scaffold, the native tissue layer, and then next layer of scaffold. In this way, the scaffold is situated to sandwich the native tissue and also minced tissue. The interfaces of the tissue will also match up so that vascularization and cell organization is facilitated.

Example 4. Processed Cells
A small 2 cm-by-2 cm piece of tissue is excised from a normal healthy bladder. The smooth muscle cell layer is then removed from the Urothelial cell layer using a scalpel. This creates two distinct tissue samples having different cell types. Each sample is put through a digestion process to isolate individual cells. Once isolated, the cells are suspended in a collagen gel.

Each cell-collagen suspension is subsequently spread on a separate 3 cm-by-3 cm square Polyglactin 910 scaffold (300 mg/cc, 1 mm thick), leaving bare 0.5 cm around the scaffold perimeter. This results in two scaffolds with two different tissue types. The two scaffolds are sandwiched together to form a four-layer composite (scaffold, cell-collagen suspension A, cell-collagen suspension B, scaffold).

Larger cuts are made in the patient's bladder in a shape that eases the placement of the implant. Each scaffold is fixed by passing sutures through a layer of scaffold, the native tissue layer, and then next layer of scaffold. In this way, the scaffold is situated to sandwich the native tissue and also minced tissue. The interfaces of the tissue will also match up so that vascularization and cell organization is facilitated.

Example 5. Tissue biopsy
A small 2 cm-by-2 cm piece of tissue is excised from a normal healthy bladder. Small cuts are made in the tissue at various intervals, and the tissue then stretched into a 3 cm-by-3 cm sample, the stretching creating voids where the cuts have been made. Biocompatible filler such as fibrin glue, may be placed inside the void to maintain sample shape and facilitate healing.

The processed sample is then sandwiched between a 4 cm-by-4 cm square Polyglactin 910 scaffold (300 mg/cc, 1 mm thick), leaving bare 0.5 cm around the scaffold perimeter. The construction creates a 3-layered composite (scaffold, processed tissue, scaffold).

Larger cuts are made in the patient's bladder in a shape that eases the placement of the implant. Each scaffold is fixed by passing sutures through a layer of scaffold, the native tissue layer, and then next layer of scaffold. In this way, the scaffold is situated to sandwich the native tissue and also minced tissue. The interfaces of the tissue will also match up so that vascularization and cell organization is facilitated.

## Claims

1. A device for augmenting or regenerating tissue comprising:
(a) first and second layers of biocompatible, biodegradable scaffolding;
(b) at least one layer of autologous tissue disposed between and substantially in contact with the first and second scaffolding layers.

2. The device of claim 1 which includes a means of fixing the device in place.

3. The device of claim 2 in which the fixation means is a suture.

4. The device of claim 1 in which the autologous tissue layer is a cellular layer.

5. The device of claim 1 in which the biocompatible scaffold layer is comprised of a material selected from the group consisting of natural polymers, synthetic polymers, bioactive glasses, ceramics, and hydrogels.

6. The device of claim 5 in which the scaffold layer is Polyglactin 910.

7. The device of claim 1 which includes a pharmaceutical agent.

8. The device of claim 7 in which the pharmaceutical agent comprises a biological factor.

9. The device of claim 8 in which the biological factor is an antibody, growth factor, hormone, genetically modified cell, or cytokine.

10. The device of claim 7 in which the pharmaceutical is a drug.

11. The device of claim 10 in which the drug is an antibiotic, analgesic, or anti-inflammatory agent.

12. The device of claim 1 which includes a biocompatible filler material.

13. The device of claim 12 in which the filler is fibrin.

14. The device of claim 1 which includes a second layer of cellular tissue that is of a different cell type than the first cell layer.

15. The device of claim 14 in which the second cell layer is separated from the first cell layer by a third layer of biocompatible scaffolding.

16. The device of claim 1 in which the autologous tissue is resected tissue.

17. The device of claim 1 in which the tissue is bladder tissue.
